# EUROPEAN PATENT APPLICATION

(11) **EP 2 172 562 A2**
(43) Date of publication of application: **07.04.2010**
(21) Application number: 08787638.9
(22) Date of filing: 27.06.2008
(51) Int. Cl.: C12Q 1/68

(54) **METHOD FOR THE INTERNALIZATION OF NON-INVASIVE BACTERIA IN EUKARYOTE CELLS**

(30) Priority: 29.06.2007 ES 200701836
(71) Applicant: Universidad del Pais Vasco, 48940 Leioa Vizcaya (ES)
(72) Inventor: OSTOLAZA ETXABE, Helena, E-48940 Leioa (ES); MARTÍN PLÁGARO, César, E-48940 Leioa (ES); GOÑI URCELAY, Félix, María, E-48940 Leioa (ES)
(74) Representative: ABG Patentes, S.L.
(86) International application number: PCT/ES2008/000459
(87) International publication number: WO 2009/004102

(57) **Abstract**

The present invention relates to the use of adenylate cyclase toxin (ACT), or a functionally equivalent variant thereof, as an inducing agent for the internalization of non-invasive bacteria in eukaryotic cells. Due to ACT, said non-invasive bacterium can move through the plasma membrane of a eukaryotic cell and transfer plasmid DNA to said cell, which is useful for releasing or introducing molecules of therapeutic interest, for example, antigens, in the interior of the eukaryotic cell, thereby triggering the immune response.

SEQUENCE LISTING

<110>  Universidad del País Vasco

<120>  METHOD FOR THE INTERNALIZATION OF NON-INVASIVE BACTERIA IN
                EUKARYOTIC CELLS

<130>  P1683EPPC

<140>  EP 08787638.9
<141>  2008-06-27

<150>  P200701836
<151>  2007-06-29

<160>  1

<170>  PatentIn version 3.3

<210>  1
<211>  1706
<212>  PRT
<213>  Bordetella pertussis

<400>  1

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Arg Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Ser Gly Arg Ser Lys Phe Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460

Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro
545                 550                 555                 560

Ala Gly Gln Lys Ala Ala Ala Gly Ala Glu Ile Ala Leu Gln Leu Thr
                565                 570                 575

Gly Gly Thr Val Glu Leu Ala Ser Ser Ile Ala Leu Ala Leu Ala Ala
            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala
625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Ala Ala Val Ser Ile Ala Ala Ala Ala
        675                 680                 685

Ser Val Val Gly Ala Pro Val Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720

Leu Ala Asn Asp Tyr Ala Arg Lys Ile Asp Glu Leu Gly Gly Pro Gln
                725                 730                 735

Ala Tyr Phe Glu Lys Asn Leu Gln Ala Arg His Glu Gln Leu Ala Asn
            740                 745                 750

Ser Asp Gly Leu Arg Lys Met Leu Ala Asp Leu Gln Ala Gly Trp Asn
        755                 760                 765

Ala Ser Ser Val Ile Gly Val Gln Thr Thr Glu Ile Ser Lys Ser Ala
    770                 775                 780

Leu Glu Leu Ala Ala Ile Thr Gly Asn Ala Asp Asn Leu Lys Ser Val
785                 790                 795                 800

Asp Val Phe Val Asp Arg Phe Val Gln Gly Glu Arg Val Ala Gly Gln
                805                 810                 815

Pro Val Val Leu Asp Val Ala Ala Gly Gly Ile Asp Ile Ala Ser Arg
            820                 825                 830

Lys Gly Glu Arg Pro Ala Leu Thr Phe Ile Thr Pro Leu Ala Ala Pro
        835                 840                 845

Gly Glu Glu Gln Arg Arg Arg Thr Lys Thr Gly Lys Ser Glu Phe Thr
    850                 855                 860

Thr Phe Val Glu Ile Val Gly Lys Gln Asp Arg Trp Arg Ile Arg Asp
865                 870                 875                 880

Gly Ala Ala Asp Thr Thr Ile Asp Leu Ala Lys Val Val Ser Gln Leu
                885                 890                 895

Val Asp Ala Asn Gly Val Leu Lys His Ser Ile Lys Leu Asp Val Ile
            900                 905                 910

Gly Gly Asp Gly Asp Asp Val Val Leu Ala Asn Ala Ser Arg Ile His
        915                 920                 925

Tyr Asp Gly Gly Ala Gly Thr Asn Thr Val Ser Tyr Ala Ala Leu Gly
    930                 935                 940

Arg Gln Asp Ser Ile Thr Val Ser Ala Asp Gly Glu Arg Phe Asn Val
945                 950                 955                 960

Arg Lys Gln Leu Asn Asn Ala Asn Val Tyr Arg Glu Gly Val Ala Thr
                965                 970                 975

Gln Thr Thr Ala Tyr Gly Lys Arg Thr Glu Asn Val Gln Tyr Arg His
            980                 985                 990

Val Glu Leu Ala Arg Val Gly Gln  Leu Val Glu Val Asp  Thr Leu Glu
        995                 1000                1005

His Val Gln His Ile Ile Gly Gly Ala Gly Asn Asp Ser Ile Thr
    1010                1015                1020

Gly Asn Ala His Asp Asn Phe Leu Ala Gly Gly Ser Gly Asp Asp
    1025                1030                1035

Arg Leu Asp Gly Gly Ala Gly Asn Asp Thr Leu Val Gly Gly Glu
    1040                1045                1050

Gly Gln Asn Thr Val Ile Gly Gly Ala Gly Asp Asp Val Phe Leu
    1055                1060                1065

Gln Asp Leu Gly Val Trp Ser Asn Gln Leu Asp Gly Gly Ala Gly
    1070                1075                1080

Val Asp Thr Val Lys Tyr Asn Val His Gln Pro Ser Glu Glu Arg
    1085                1090                1095

Leu Glu Arg Met Gly Asp Thr Gly Ile His Ala Asp Leu Gln Lys
    1100                1105                1110

Gly Thr Val Glu Lys Trp Pro Ala Leu Asn Leu Phe Ser Val Asp
    1115                1120                1125

His Val Lys Asn Ile Glu Asn Leu His Gly Ser Arg Leu Asn Asp
    1130                1135                1140

Arg Ile Ala Gly Asp Asp Gln Asp Asn Glu Leu Trp Gly His Asp
    1145                1150                1155

Gly Asn Asp Thr Ile Arg Gly Arg Gly Gly Asp Asp Ile Leu Arg
    1160                1165                1170

Gly Gly Leu Gly Leu Asp Thr Leu Tyr Gly Glu Asp Gly Asn Asp
    1175                1180                1185

Ile Phe Leu Gln Asp Asp Glu Thr Val Ser Asp Asp Ile Asp Gly
    1190                1195                1200

Gly Ala Gly Leu Asp Thr Val Asp Tyr Ser Ala Met Ile His Pro
    1205                1210                1215

Gly Arg Ile Val Ala Pro His Glu Tyr Gly Phe Gly Ile Glu Ala
    1220                1225                1230

Asp Leu Ser Arg Glu Trp Val Arg Lys Ala Ser Ala Leu Gly Val
    1235                1240                1245

Asp Tyr Tyr Asp Asn Val Arg Asn Val Glu Asn Val Ile Gly Thr
    1250                1255                1260

Ser Met Lys Asp Val Leu Ile Gly Asp Ala Gln Ala Asn Thr Leu
    1265                1270                1275

Met Gly Gln Gly Gly Asp Asp Thr Val Arg Gly Gly Asp Gly Asp
    1280                1285                1290

Asp Leu Leu Phe Gly Gly Asp Gly Asn Asp Met Leu Tyr Gly Asp
    1295                1300                1305

Ala Gly Asn Asp Thr Leu Tyr Gly Gly Leu Gly Asp Asp Thr Leu
    1310                1315                1320

Glu Gly Gly Ala Gly Asn Asp Trp Phe Gly Gln Thr Gln Ala Arg
    1325                1330                1335

Glu His Asp Val Leu Arg Gly Gly Asp Gly Val Asp Thr Val Asp
    1340                1345                1350

Tyr Ser Gln Thr Gly Ala His Ala Gly Ile Ala Ala Gly Arg Ile
    1355                1360                1365

Gly Leu Gly Ile Leu Ala Asp Leu Gly Ala Gly Arg Val Asp Lys
    1370                1375                1380

Leu Gly Glu Ala Gly Ser Ser Ala Tyr Asp Thr Val Ser Gly Ile
    1385                1390                1395

Glu Asn Val Val Gly Thr Glu Leu Ala Asp Arg Ile Thr Gly Asp
    1400                1405                1410

Ala Gln Ala Asn Val Leu Arg Gly Ala Gly Gly Ala Asp Val Leu
    1415                1420                1425

Ala Gly Gly Glu Gly Asp Asp Val Leu Leu Gly Gly Asp Gly Asp
    1430                1435                1440

Asp Gln Leu Ser Gly Asp Ala Gly Arg Asp Arg Leu Tyr Gly Glu
    1445                1450                1455

Ala Gly Asp Asp Trp Phe Phe Gln Asp Ala Ala Asn Ala Gly Asn
    1460                1465                1470

Leu Leu Asp Gly Gly Asp Gly Arg Asp Thr Val Asp Phe Ser Gly
    1475                1480                1485

Pro Gly Arg Gly Leu Asp Ala Gly Ala Lys Gly Val Phe Leu Ser
    1490                1495                1500

Leu Gly Lys Gly Phe Ala Ser Leu Met Asp Glu Pro Glu Thr Ser
    1505                1510                1515

Asn Val Leu Arg Asn Ile Glu Asn Ala Val Gly Ser Ala Arg Asp
    1520                1525                1530

Asp Val Leu Ile Gly Asp Ala Gly Ala Asn Val Leu Asn Gly Leu
    1535                1540                1545

Ala Gly Asn Asp Val Leu Ser Gly Gly Ala Gly Asp Asp Val Leu
    1550                1555                1560

Leu Gly Asp Glu Gly Ser Asp Leu Leu Ser Gly Asp Ala Gly Asn
    1565                1570                1575

Asp Asp Leu Phe Gly Gly Gln Gly Asp Asp Thr Tyr Leu Phe Gly
    1580                1585                1590

Val Gly Tyr Gly His Asp Thr Ile Tyr Glu Ser Gly Gly Gly His
    1595                1600                1605

Asp Thr Ile Arg Ile Asn Ala Gly Ala Asp Gln Leu Trp Phe Ala
    1610                1615                1620

Arg Gln Gly Asn Asp Leu Glu Ile Arg Ile Leu Gly Thr Asp Asp
    1625                1630                1635

Ala Leu Thr Val His Asp Trp Tyr Arg Asp Ala Asp His Arg Val
    1640                1645                1650

Glu Ile Ile His Ala Ala Asn Gln Ala Val Asp Gln Ala Gly Ile
    1655                1660                1665

Glu Lys Leu Val Glu Ala Met Ala Gln Tyr Pro Asp Pro Gly Ala
    1670                1675                1680

Ala Ala Ala Ala Pro Pro Ala Ala Arg Val Pro Asp Thr Leu Met
    1685                1690                1695

Gln Ser Leu Ala Val Asn Trp Arg
    1700                1705

## Description

### Field of the Invention

The invention generally relates to the internalization of non-invasive bacteria in eukaryotic cells for therapeutic and/or prophylactic purposes; the invention particularly relates to the use of adenylate cyclase toxin (ACT) or a functionally equivalent variant thereof, as an inducing agent for the internalization of non-invasive bacteria in eukaryotic cells.

### Background of the Invention

The principle of vaccination is to induce a long-term protective immune response in the host against a virulent microorganism. The purpose of the vaccines is therefore to prevent and control future infections.

Many pathogenic bacteria are capable of escaping from the immune system by inducing their own internalization in mammal cells and efficiently replicating therein. The entry of said bacteria to the cell interior is mediated by typical phagocytosis when the cells are professional phagocytes, or by induced phagocytosis in the case of non-phagocytic cells (epithelial cells, hepatocytes, fibroblasts and endothelial cells).

Taking advantage of this property, the bactofection technique has been developed as a tool facilitating genetic vaccination. Bactofection consists of the transfer of plasmid DNA to mammal cells mediated by intracellular bacteria. These bacteria are characterized in that for their development and replication they need to be in the interior of a cell. Thus, the use of intracellular bacteria has become a direct method for DNA transport which achieves the expression of heterologous proteins (protein antigens, toxins or enzymes) capable of triggering both humoral and cellular immune responses.

The strategy of bactofection is based on the susceptibility of transforming intracellular pathogenic bacteria with eukaryotic expression vectors. Attenuated strains of these bacteria can thus transport said plasmids to the antigen-presenting cells (APC). Once internalized, the bacteria alter the characteristics of the phagosomal compartment preventing the fusion with lysosomes and thus assuring their survival. The capacity, in many of them, of lysing the phagosome and replicating in the cytosol has also been described. Experiments with *Listeria monocytogenes* have shown that plasmid DNA is transported to the cell cytosol after a partial self-destruction of the bacterium. Once in the cytosol, the DNA can be transported to the cell nucleus and, the antigens encoded by the plasmid are thus expressed in the antigen-presenting cell.

Although the mechanism of transporting plasmid DNA from the phagolysosome to the cell nucleus is not known, it has been shown that these attenuated strains can be used as vaccine carriers not only for rodents, but also for primate cells and for human dendritic cells.

One of the main attractions of these bacterial vectors is their potential for being administered orally, in addition to allowing the induction of immune response in the mucosa, essential in those diseases caused by pathogens with an entryway into the organism through the mucosae.

Another alternative mechanism of vaccination used in recent years is based on the recombinant antigen-presenting bacterial systems. In these models, the heterologous antigens synthesized by bacteria are only accessible after the disintegration of the bacterium. Alternatives for exporting antigens to the cytoplasm have been developed, including the use of the MalE and OmpA export signals, or fusion with the LT-B toxin of *Escherichia coli*. Two heterologous antigen secretion systems by Gram negative bacteria have also been described, including the type III secretion system, which allows the "injection" of the antigen into the target cell and the AIDA autotransporter system. However, many of these systems can only carry small peptides of the heterologous antigens. To solve the antigen size problem, the use of the hemolysin (HlyA) secretion system has begun since it seems that it does not involve any limitation in the size of the transported antigens. This transporter allows the active secretion of large heterologous antigens by attenuated bacteria, which has lead to the development of a large number of live recombinant vaccines. Many heterologous proteins presented by means of this route induce humoral and/or cellular immune responses to the organism host. These immune responses show that there is an active expression and secretion of the heterologous antigens *in vivo.*

Bacterial transporters can colonize the cell surface, the specialized phagosome of the antigen-presenting cells (APC) or the cytosol of the infected cells and transport one and the same antigen to different compartments of the APC, thus triggering different immune responses against one and the same antigen.

In view of the foregoing, there is a need to provide an alternative mechanism to the use of attenuated pathogen strains in these bacterial vector vaccines due to the risk they involve since they are formed by live microorganisms. In fact, one of the main drawbacks of these vaccines is the possible reversion towards the pathogenicity, or that these microorganisms maintain their pathogen activity. Despite the high degree of attenuation which is achieved in this type of bacteria, their application can be compromised in patients with primary or secondary immunodeficiencies or who have an immunosuppression condition caused by drugs or opportunistic diseases, as well as in pregnant women.

Adenylate cyclase toxin (ACT) is a protein with the capacity of being internalized in the cytoplasm of eukaryotic cells and, after its activation by calmodulin, catalyzing the uncontrolled transformation of adenosine triphosphate (ATP) into cyclic adenosine monophosphate (cAMP) causing the intoxication of said eukaryotic cell. Said ACT is a single example of an enzymatically active toxin capable of translocating directly through the plasma membrane of a cell, without receptor-mediated endocytosis being necessary. The molecular mechanism whereby the translocation of the catalytic domain to the cytoplasm of the cells takes place is currently unknown. It is known that in some cell types a negative membrane potential is necessary, although the toxin can efficiently invade cells with a low membrane potential, as in the case of erythrocytes, which furthermore lack intracellular membrane trafficking. In this case, it is believed that the penetration into the interior of the erythrocyte is initiated with an unspecific adsorption of the toxin on the surface of the cell, followed by direct insertion and translocation of the 40 kDa segment through the membrane, although the molecular events taking place in the process are not known. Adenylate cyclase is, like α-hemolysin of *E. coli,* a toxin with broad cell specificity. It has recently been described that integrin α_{M}β2 (CD11b/CD18) constitutes the specific receptor for ACT in leukocytes, as well as the possible use of said ACT in the delivery of epitopes of T cells towards the presentation routes of the major histocompatibility complex class I or class II.

### Summary of the Invention

It has been surprisingly found now that when adenylate cyclase toxin (ACT) is bound to the outer membrane of a non-invasive bacterium, said bacterium can be internalized and invade a eukaryotic cell.

Therefore, in one aspect, the invention relates to the use of ACT, or a functionally equivalent variant thereof, as an inducing agent for the internalization of non-invasive bacteria in eukaryotic cells.

In another aspect, the invention relates to a non-invasive bacterium comprising ACT or a functionally equivalent variant thereof bound to its membrane. In a particular embodiment, said bacterium furthermore comprises
(i) a polynucleotide encoding a heterologous polypeptide of interest, or
(ii) a gene construct comprising a polynucleotide according to (i), or
(iii) a plasmid comprising a gene construct according to (ii) or
(iv) a heterologous polypeptide of interest.

In another aspect, the invention relates to a pharmaceutical composition comprising a non-invasive bacterium comprising ACT or a functionally equivalent variant thereof provided by this invention bound to its membrane, and a pharmaceutically acceptable carrier.

Additional aspects of the present invention include the different uses of said non-invasive bacterium comprising ACT or a functionally equivalent variant thereof bound to its membrane, such as the use of said bacterium as a medicament and the use of said bacterium in the preparation of a vaccine.

In another aspect, the invention relates to a process for obtaining a non-invasive bacterium comprising ACT or a functionally equivalent variant thereof bound to its membrane, comprising incubating a non-invasive bacterium with ACT, or with a functionally equivalent variant thereof, or alternatively transforming a non-invasive bacterium with a polynucleotide encoding the ACT or a functionally equivalent variant thereof.

Finally, the present invention relates to a method for inducing the internalization of non-invasive bacteria in eukaryotic cells comprising:
(a) incubating a non-invasive bacterium in the presence of ACT, or a functionally equivalent variant thereof, or alternatively transforming the non-invasive bacterium with a polynucleotide encoding ACT, or a functionally equivalent variant thereof, and
(b) contacting the bacterium obtained in paragraph (a) with a eukaryotic cell culture.

### Brief Description of the Drawings

Figure 1 shows the intracellular cAMP levels in CHO cells after the treatment with different ACT concentrations (0.5-20 µg/ml) or with *E. coli* bacteria coated with ACT at different concentrations (10-100 µg).

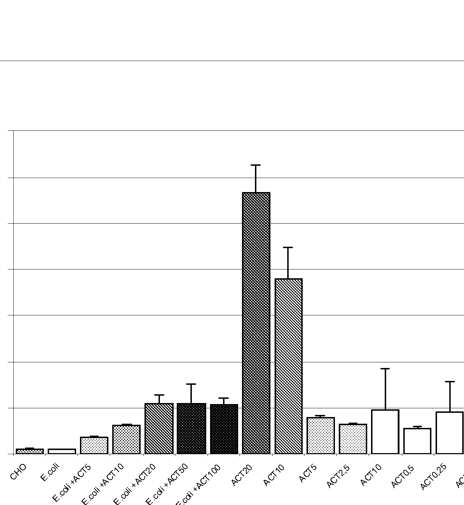

Figure 2 shows the reorganization of the cytoskeleton of CHO cells induced by ACT. Figure 2a shows the control CHO cells stained with Alexa Fluoro488 phalloidin and 4',6-diamidino-2-phenylindole (DAPI) to see the F-actin fibers and DNA, respectively. Figure 2b shows CHO cells treated with soluble ACT at a concentration of 20 µg/ml. Figure 2c shows CHO cells treated with soluble ACT at a concentration of 2 µg/ml. Figure 2d shows CHO cells treated with *E. coli* coated with ACT. The amount of ACT bound to the cell surface is 0.55 µg toxin/10⁸ bacteria. Primary anti-ACT monoclonal antibodies and secondary antibodies bound to Texas Red were used to detect the bacteria coated with ACT.
Figure 3 corresponds to scanning electron microscope pictures in which the interaction of the bacteria coated with ACT with the CHO cells is seen. The co-incubation of the CHO cells and *E. coli* coated with ACT results in the formation of structures similar to the pseudopodia surrounding the bacteria prior to their internalization.
Figure 4 shows the different steps of the internalization of bacteria coated with ACT in CHO cells. Figure 4a shows the contact of the bacteria with the cell membrane. Figure 4b shows a strong adhesion of the bacterium to the cell membrane. Figure 4c shows the phagocytosis of the bacterium. Figure 4d shows an invasive bacterium surrounded by membranous vesicles.
Figure 5 shows the interaction of ACT with the actin detected by means of dot blotting technique.
Figure 6 shows pyrene-labeled actin polymerization induced by ACT. The kinetics of polymerization is detected by the increase of fluorescence occurring when polymerizing pyrene actin.
Figure 7 are electron microphotographs corresponding to control F-actin and to actin filaments obtained by means of the incubation of G-actin with ACT.
Figure 8 shows large halos of polymerized actin surrounding bacteria coated with ACT and incubated with G-actin.

### Detailed Description of the Invention

In one aspect, the present invention relates to the use of adenylate cyclase toxin (ACT), or a functionally equivalent variant thereof, as an inducing agent for the internalization of non-invasive bacteria in eukaryotic cells.

As it is used in this description, the term "adenylate cyclase toxin" or "ACT" (also known as CyaA) relates to a protein which catalyzes the transformation of ATP into cAMP and it is furthermore capable of translocating directly through the plasma membrane of a eukaryotic cell. In a particular embodiment, said ACT is derived from a microorganism of the *Bordetella sp*. genus, e.g., *B. pertussis, B. parapertussis, B. bronchiseptica,* etc., or of related molecules of other bacteria. Related molecules include proteins of other bacteria with sequences homologous to those of ACT. In another particular embodiment, said ACT has the amino acid sequence shown in SEQ ID NO: 1, which corresponds to the sequence of *B. pertussis* ACT (also referred to as CyaA) accessible in the Swiss-Prot database under code P15318 or in the NCBI database under code NP_879578. Likewise, in another particular embodiment, the ACT corresponds to the recombinant protein expressed in *E. coli* K12 XL-1blue transformed with the plasmid pT7CACT1 obtained according to Osicka *et al.* (Osicka, RA et al. 2000 Infect. Immun 68:247-256*)* or Martin et al. (Martin et al., 2004. J. Bacteriol 186:3760-3765). The CyaA DNA sequence lacks polymorphisms between the *B. pertussis* isolates (Packard ER, et al., 2004, J. Med. Microbiol. Vol. 53: 355-365) and has high homology between the different *Bordetella sp.* species with an identity greater than 97% between *B. pertussis* and *B. bronchiseptica* or *B. parapertussis* (Parkhill, J. et al. 2003. Nat. Genet. Vol. 35: 32-40).

As it is used herein, the term "protein" relates to an amino acid molecular chain, bound by covalent or non-covalent bonds. The term furthermore includes all the physiologically relevant post-translational chemical modification forms, for example, glycosylation, phosphorylation or acetylation, provided that the capacity of inducing the internalization of non-invasive bacteria in eukaryotic cells is maintained.

As it is used herein, the expression "functionally equivalent variant" relates to a protein the amino acid sequence of which (i) is substantially homologous to the amino acid sequence of a certain ACT and (ii) maintains at least one of the activities of said ACT, for example, the capacity of catalyzing the transformation of ATP into cAMP, the capacity of acting as an inducing agent for the internalization of non-invasive bacteria in eukaryotic cells, etc., preferably at least the latter.

An amino acid sequence is substantially homologous to a certain amino acid sequence when it has a degree of identity of at least 70%, advantageously of at least 75%, typically of at least 80%, preferably of at least 85%, more preferably of at least 90%, still more preferably of at least 95%, 97%, 98% or 99%, with respect to said certain amino acid sequence. The degree of identity between two amino acid sequences can be determined by conventional methods, for example, by means of standard sequence alignment algorithms known in the state of the art, such as, for example BLAST [Altschul S.F. et al. Basic local alignment search tool. J Mol Biol. 1990 Oct 5; 215(3):403-10].

The capacity of ACT, or of a functionally equivalent variant thereof, of acting as an inducing agent for the internalization of non-invasive bacteria in a eukaryotic cell can be evaluated by any conventional method, for example, by means of a protection assay with antibiotics such as that described in Example 1 (section 1.9 of Materials and Methods).

The person skilled in the art understands that the mutations in the nucleotide sequence of ACT which give rise to conservative substitutions of amino acids in positions that are not critical for the functionality of the protein are evolutionarily neutral mutations which do not affect its overall structure or functionality. Said variants fall within the scope of the present invention. Those functionally equivalent variants of a certain ACT having insertions, deletions or modifications of one or more amino acids with respect to said certain ACT, and furthermore preserve the capacity of acting as an inducing agent for the internalization of non-invasive bacteria in eukaryotic cells are also within the scope of the invention.

Therefore, as it is used herein, the term "functionally equivalent variant" also includes any functionally equivalent fragment of an ACT. The term "fragment" relates to a peptide comprising a portion of a protein. In this case, a functionally equivalent fragment of an ACT is a peptide or protein comprising a portion of an ACT and maintaining the capacity of acting as an inducing agent for the internalization of non-invasive bacteria in eukaryotic cells.

Virtually any ACT or functionally equivalent variant thereof can be used for putting the present invention into practice; nevertheless, in a particular embodiment, said ACT is an ACT isolated from *Bordetella sp.,* e.g., *B. pertussis, B. brochiseptica, B. parapertussis,* etc. In a specific embodiment, said ACT has the amino acid sequence shown in SEQ ID NO: 1.

Said ACT can be obtained from a microorganism producing said protein in a native or recombinant form. ACT can be obtained and purified by conventional methods known by the person skilled in the art. By way of a non-limiting illustration, Example 1 describes a process for obtaining and purifying an ACT with the capacity of being internalized in eukaryotic cells and efficiently replicating therein.

In the present invention, "inducing agent for the internalization of non-invasive bacteria in eukaryotic cells" is understood as that agent which is capable of causing or inducing the entry of a non-invasive bacterium in a eukaryotic cell. By way of illustration, the use of ACT as an inducing agent for the internalization of non-invasive bacteria is aimed at those bacteria (i) which lack the capacity of being internalized in a eukaryotic cell naturally, or (ii) which, despite having said capacity of being internalized in a eukaryotic cell naturally ("invasive bacteria"), have been genetically modified so that they cannot do so.

Therefore, as it is used herein, the expression "non-invasive bacterium" relates to a bacterium which is not an invasive bacterium. Likewise, the term "invasive bacterium", relates to a bacterium which naturally has the capacity of inducing its own internalization or entry in the cytoplasm of a eukaryotic cell and efficiently replicating therein. A definition of "invasive bacterium" can be found in Bonazzi M. and Cossart, P. 2006. Bacterial entry into cells: a role for the endocytic machinery. FEBS Lett Vol.580(12):2962-2967. Assays to determine if a bacterium is invasive are widely known by the person skilled in the art, see, for example Boer, EC., et al. 1996. Cytometry. Vol. 25(4): 381-387; Burton, E.A., et al. Abl tyrosine kinases are required for infection by Shigella flexneri. The EMBO Journal. Vol. 22(20): 5471-5479; and international patent application WO 90/12867.

In another aspect, the invention relates to a bacterium, hereinafter non-invasive bacterium of the invention, comprising an ACT or a functionally equivalent variant thereof bound to its membrane. Said ACT or functionally equivalent variant thereof can be associated with the membrane of the non-invasive bacterium by coating, adhering or anchoring, for example, it can be associated with the outer surface of the membrane of the non-invasive bacterium by coating, adhering or anchoring thereon. In the examples attached to the present description, said ACT is adhered on the outer surface of the membrane of a non-invasive bacterium.

Choosing the type of non-invasive bacterium used according to the present invention will depend, among other factors, on the size of its genome, on the replicative capacity and on genetic stability. The non-invasive bacterium is preferably a non-pathogenic bacterium (i.e., it does not have sufficient metabolic capacity to cause damage, alone or in association with other factors, to a subject and cause disease) for an animal, more preferably for humans. By way of a non-limiting illustration, non-invasive bacteria according to the present invention include, for example, *Escherichia coli* and *Agrobacterium tumefaciens,* as shown in the examples attached to the present description.

As will be discussed below, the binding of said ACT to the outer membrane of the non-invasive bacterium allows the latter to be internalized in eukaryotic cells; therefore the bacterium of the invention can be used to introduce polynucleotides, plasmids and/or heterologous polypeptides of interest in eukaryotic cells.

Therefore, in a particular embodiment, the bacterium of the invention furthermore comprises
(i) a polynucleotide encoding a heterologous polypeptide of interest, or
(ii) a gene construct comprising a polynucleotide according to (i), or
(iii) a plasmid comprising a gene construct according to (ii) or
(iv) a heterologous polypeptide of interest.

As is used herein, the term "heterologous polypeptide of interest" relates to any heterologous polypeptide (e.g., peptide or protein) which is to be introduced in a eukaryotic cell, such as an antigen, a toxin, an enzyme, etc. Non-limiting illustrative examples of antigens which can be used as "heterologous polypeptides of interest" in the present invention include:
■ Peptides or proteins capable of (suitable or designed for) inducing an immune response against an infectious disease, such as an infectious disease in animals caused by pathogenic microorganisms of animals, including humans, for example, virus, bacteria, fungi and infectious parasites, relevant in human or animal health.
   The proteins or peptides capable of inducing an immune response can be recombinant proteins or peptides, identical or similar to the natural antigens of a specific microorganism.
   Non-limiting illustrative examples of infectious virus include virus of the families: Arteriviridae, Retroviridae, Picornaviridae, Calciviridae, Togaviridae, Flaviridae, Coronoviridae, Rhabdoviradae, Filoviridae, Paramyxoviridae, Orthomyxoviridae, Bungaviridae, Arenaviridae, Reoviridae, Birnaviridae, Hepadnaviridae, Parvoviridae (parvovirus), Papovaviridae, Adenoviridae, Herpesviridae, Poxviridae, Iridoviridae, etc. Examples of antigens which can be used according to the present invention include but are not limited to HIV antigens, gp120 antigen, hepatitis B surface antigen, rotavirus antigens such as VP4 and VP7, influenza virus antigens such as hemagglutinin or nucleoprotein, thymidine kinase herpes simplex antigen, etc.
   Non-limiting illustrative examples of bacteria include both Gram positive bacteria, e.g., *Pasteurella sp., Staphylococcus sp., Streptococcus sp.,* etc., and Gram negative bacteria, e.g., *Escherichia coli, Pseudomonas sp., Salmonella sp.,* etc. Specific examples of infectious bacteria include: *Helicobacter pylori, Borelia burgdorferi, Legionella pneumoplailia, Mycobacteria sp*. (e.g., M. *tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogefaes* (Streptococcus Group A), *Streptococcus agalactiae* (Streptococcus Group B), Streptococcus (viridans group), *Streptococcus faecalis, Streptococcus bovis, Streptococcus* (anaerobic species), *Streptococcus pneumoniae, Campylobacter sp., Enterococcus sp., Haemophilus influenzae, Bacillus aratracis, Corynebacterium diphtheriae, Corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponemapallidium, Treponema* pertenue, *Leptospira, Rickettsia, Actinornyces israelli, Chlamydia,* etc.
   Non-limiting illustrative examples of infectious fungi include *Cryptococcus neoformans, Histoplasma capsulatum, Coccidioides immitis, Blastomyces dermatitidis, Chlamydia trachomatis* and *Candida albicans.*
   By way of non-limiting illustration, protozoa are included among the infectious parasites, such as *Plasmodium sp.,* protozoa causing malaria, e.g. *P. falciparum, P. malariae, P. ovale, P. vivax,* etc., *Leishmania sp.,* protozoa causing leishmaniasis, e.g., *L. major, L. donovani, L. infantum, L. braziliensis, L. panamensis, L. mexicana,* etc., *Toxoplasma gondii, Schistosoma sp.,* etc., as well as parasitic nematodes, such as *Dirofilaria immitis,* etc. Examples of antigens for these parasites include *Plasmodium spp*. circumsporozoite antigen; *Plasmodium spp.* merozoite surface antigen; *Leishmania spp.* gp63, etc.
■ Peptides or proteins associated with tumors or cancers ("tumor markers") capable of (suitable or designed for) inducing an immune response against a tumor or cancer cell, therefore the heterologous polypeptide of interest can be used in the treatment of cancers by means of the stimulation of an antigen-specific immune response against a tumor antigen.
   Non-limiting illustrative examples of cancers which could be potentially treated according to the teachings of the present invention include bile duct cancer, brain cancer, breast cancer, cervical cancer, choriocarcinoma, colon cancer, endometrial cancer, esophageal cancer, stomach cancer, intraepithelial neoplasias, lymphomas, liver cancer, lung cancer (e.g., small and non-small cell lung cancer), melanoma, neuroblastomas, mouth cancer, ovarian cancer, pancreatic cancer, prostate cancer, rectal cancer, sarcomas, skin cancer, testicular cancer, thyroid cancer and renal cancer, as well as other carcinomas and sarcomas.
   A person skilled in the art can select tumor antigens or antigenic determinants for the treatment of cancers in view of the state of the art [Renkvist et al., Cancer Immunol. Immunother. 50:3-15 (2001)], said antigens and antigenic determinants being included within the scope of the present invention. Representative examples of said antigens or antigenic determinants include: Her2 (breast cancer); GD2 (neuroblastoma); EGF-R (malignant glioblastoma); CEA (medullary thyroid cancer); CD52 (leukemia); human melanoma gp100 protein; human melanoma melan-A/MART-1 protein; tyrosinase; NA17-A nt protein; MAGE-3 protein; p53 protein; HPV16E7 protein; and antigenic fragments of said peptides or proteins.
■ Peptides or proteins capable of (suitable or designed for) inducing an immune response against an allergen.
   As it is used in this description, the term "allergen" relates to a peptide or protein to which a subject is sensitive and causes an immune reaction, for example, allergen extracts of pollens, allergen extracts of insects, allergen extracts of food or food products, components present in saliva, insect claws or stings which induce a sensitivity reaction in a subject, components present in plants which induce a sensitivity reaction in a subject, etc. Non-limiting illustrative examples of allergens include protein extracts of pollens, e.g., of *Lolium perenne, Poa pratense, Phleum pratense, Cynodon dactylon, Festuca pratensis, Dactylis glomerata, Secale cereale, Hordeum vulgare, Avena sativa, Triticum sativa, Artemisia vulgaris, Chenopodium album, Plantago lanceolata, Taraxacum vulgare, Parietaria judaica, Salsola kali, Urtica dioica, Olea europea, Platanus sp., Cupressus sp.,* etc.; protein extracts of insects, e.g., of *Dermatophagoides pteronyssinus, Dermatophagoides farinae, Acarus siro, Blomia tropicalis, Euroglyphus maynei, Glyciphagus domesticus, Lepidoglyphus destructor, Tyrophagus putrescentiae,* etc.; protein extracts of fungi or of animal dander, e.g., *Penicillium sp., Alternaria alternata, Cladosporium herbarum,* dog dander, cat dander, horse dander, etc.; protein extracts of food or food products, etc.
■ Peptides or proteins capable of (suitable or designed for) inducing a improved response against an autoantigen. As it is used herein, the term "autoantigen" relates to peptides or proteins encoded by the DNA of the subject and products generated by proteins or RNA encoded by the DNA of the subject. Examples of autoantigens are described in WO 02/56905.

The heterologous polypeptide of interest is encoded by a polynucleotide which, in turn, can form part of a gene construct. Said polynucleotide or gene construct comprising it can be integrated in the genome of the bacterium of the invention, such that it is endogenously expressed and replicated, or it can be comprised in a vector or plasmid, such that it can be replicated and expressed independently of the genome of the bacterium of the invention. In the event that said polynucleotide or gene construct is to be inserted in the genome of the bacterium of the invention, said insertion is commonly performed by means of gene recombination techniques well known in the state of the art.

Said gene construct comprising the polynucleotide encoding a heterologous polypeptide of interest can be obtained by means of the use of techniques widely known in the state of the art [Sambrook et al., 2001. "Molecular cloning: a Laboratory Manual", 3rd ed., Cold Spring Harbor Laboratory Press, N.Y., Vol. 1-3]. The gene construct can incorporate an operatively bound control or regulating sequence of the expression of the nucleotide sequence encoding for the heterologous polypeptide of interest, thus forming an expression cassette.

As it is used in this description, the expression "operatively bound" means that the polypeptides encoded by the polynucleotide are expressed in the correct reading frame under the control of the control or regulatory sequences of the expression. Multiple expression cassettes can be used according to the present invention such that they express any combination of viral, bacterial, parasitic genes, or synthetic genes encoding all or fragments of any of the combinations of antigens previously described. The expression cassettes can also be eukaryotic, such that they encode a therapeutic agent for animal cells. For example, the expression cassette can encode a tumor-specific antigen, transplant antigen or an autoimmune antigen or fragment thereof.

Alternatively, the eukaryotic expression cassette can encode synthetic genes, encoding a tumor-specific antigen, transplant antigen or an autoimmune antigen or fragment thereof. Examples of tumor-specific antigens include TAG-72 and CEA prostate-specific antigen, MAGE-1 and tyrosinase. Examples of transplant antigens include but are not limited to the T cell CD3 receptor. Likewise, the eukaryotic expression cassettes can encode immunoregulatory molecules. Said molecules include but are not limited to growth factors and cytokines such as IL-2, IL-4, L-5, IL-6, IL-10, IL-12 or IFN-γ.

Control sequences are sequences which control and regulate the transcription and, where appropriate, the translation of messenger RNA into the heterologous polynucleotide of interest. Said control sequences include promoter sequences, sequences encoding transcriptional regulators, ribosome binding sequences (RBS) and/or transcription terminator sequences; and can be functional in prokaryotic cells and organisms, such as for example, bacteria, and/or can be functional in eukaryotic cells and organisms, such as for example, insect cells, plant cells, mammal cells, etc.

Advantageously, said gene construct furthermore comprises a marker or gene encoding a motif or a phenotype which allows selecting the host cell transformed with said construct.

Said gene construct can be inserted in a suitable vector, hereinafter vector of the invention, such as a plasmid. Therefore, said plasmid comprises the polynucleotide encoding the heterologous polypeptide of interest or the gene construct comprising said polynucleotide. The choice of the vector will depend on the host cell in which it will be subsequently introduced. By way of illustration, the plasmid in which said polynucleotide or gene construct is introduced can be a plasmid which, upon being introduced in a host cell, is integrated or not in the genome of said cell. Obtaining said vector/plasmid can be performed by conventional methods known by persons skilled in the art [Sambrook *et al.,* 2001, cited above]. Said recombinant vector/plasmid is a vector useful for transforming non-invasive bacteria. As has been previously mentioned, the vector of the invention can be a plasmid which is preferably a circular DNA molecule which can be replicated independently of the genome of the cell. Said plasmid can additionally comprise antibiotic resistance genes to select those cells which have incorporated the plasmid, a promoter, generally viral, a transcription terminator and other elements widely known by the person skilled in the art.

In another aspect, the invention relates to a pharmaceutical composition, hereinafter, pharmaceutical composition of the invention, comprising the bacterium of the invention and a pharmaceutically acceptable vehicle. Generally, the dose of bacteria of the invention to be used in the pharmaceutical composition of the invention will vary between 10³ and 10¹¹ cfu (colony forming units), more preferably between 10⁵ and 10⁹ cfu.

Additionally, the pharmaceutical composition of the invention can contain an adjuvant for the purpose of increasing the protective immune response against the antigen or antigens which are administered to the subject.

The term "subject" relates to a member of a mammal animal species and includes but is not limited to a domestic animal, a primate and a human; the subject is preferably a male or female human being of any race or age.

The dose of the pharmaceutical composition of the invention which will be administered to the subject will depend on many factors, including the characteristics of the bacterium of the invention used in the preparation of the pharmaceutical composition, the clinical condition of the subject, the disease to be treated, etc. For its administration to the subject, the pharmaceutical composition of the invention will include pharmaceutical acceptable carriers and excipients depending on the selected pharmaceutical dosage form and on the chosen administration route. By way of a non-limiting illustration, the pharmaceutical composition of the invention can be administered in the form of a suspension, etc., suitable for its administration through any suitable administration route, for example, by parenteral route, oral route, etc.

The pharmaceutical composition of the invention can be prepared by conventional methods known by the person skilled in the art. A review of the different dosage forms for the administration of drugs and their preparation can be found in the book "Tratado de Farmacia Galénica", by C. Faulí i Trillo, 10th Edition, 1993, Luzán 5, S.A. de Ediciones.

Due to the presence of ACT, the bacterium of the invention can move through the plasma membrane of a eukaryotic cell and transfer plasmid DNA to said cell. If a polynucleotide encoding a heterologous polypeptide of interest (as has been previously defined) has been introduced previously (for example, by genetic manipulation) in said plasmid DNA, then the bacterium of the invention is useful for releasing or introducing molecules of therapeutic interest in the interior of the eukaryotic cell.

Therefore, in one aspect the present invention is aimed at the use of the bacterium of the invention as a medicament.

In another aspect, the invention relates to the use of the bacterium of the invention for gene therapy.

Taking advantage of the property of the bacterium of the invention of being internalized in eukaryotic cells, the bacterium of the invention can be used as a tool to facilitate genetic vaccination, i.e., the use of the bacterium of the invention to transport DNA encoding the heterologous polypeptides of interest (protein antigens, toxins or enzymes) capable of triggering both humoral and cellular immune responses.

Therefore, in another aspect, the invention relates to the use of the bacterium of the invention in the preparation of a vaccine, hereinafter, vaccine of the invention.

The vaccines can be prepared as injectable vaccines either as liquid solutions or as suspensions; solid forms suitable for dissolution or suspension in liquid before the injection can also be prepared. In the event that the bacterium of the invention is to remain alive, the preparation of the vaccine with the components suitable for assuring the survival of the bacterium of the invention has to be taken into account.

Thus, the vaccine of the invention can be parenterally administered by means of injection both subcutaneously and intramuscularly.

In addition, the vaccine of the invention can contain an adjuvant which aids to enhance the immune response. Processes for achieving a coadjuvant effect for the vaccine include (i) the use of agents such as aluminum (alum) phosphate or hydroxide, commonly used as a solution of 0.05 to 0.1% phosphate buffer saline, (ii) mixture with synthetic sugar polymers (Carbopol) used as 0.25% solution and (iii) aggregation of the protein in the vaccine by means of heat treatment with temperatures varying between 70°C and 101°C for periods of 30 seconds and 2 minutes respectively. Other possibilities involve the use of immunomodulatory substances such as lymphokines (for example, INF-γ, IL-2 and IL-12) or synthetic INF-γ inducers such as poly I:C in combination with the previously mentioned coadjuvants. Virtually any adjuvant that can be administered by the chosen administration route can be used.

The vaccine provided by this invention can contain one or more heterologous polypeptides of interest. In a particular embodiment, said vaccine contains a single heterologous polypeptide of interest. In another particular embodiment, said vaccine contains two or more different heterologous polypeptides of interest.

The amount of antigen in each dose of vaccine is selected as an amount which induces an immunoprotective response without important adverse side effects in typical vaccines. Such amount will vary depending on the specific immunogens used.

In a particular embodiment, the use of the bacterium of the invention is aimed at the preparation of a vaccine for an immunization process by means of bactofection.

In another particular embodiment, the use of the bacterium of the invention is aimed at the preparation of a vaccine for an immunization process by means of an antigen-presenting bacterial system.

The bacterium of the invention can be obtained by any method known by the person skilled in the art, such as for example, the incubation of the non-invasive bacterium with ACT, or a functionally equivalent variant thereof, or the transformation of the non-invasive bacterium with the polynucleotide encoding ACT, or a functionally equivalent variant thereof.

Therefore, in another aspect, the invention relates to a process for obtaining the bacterium of the invention, hereinafter process of the invention, comprising incubating a non-invasive bacterium with an ACT, or a functionally equivalent variant thereof; or alternatively, transforming a non-invasive bacterium with a polynucleotide encoding the ACT, or a functionally equivalent variant thereof.

In a particular embodiment of the process of the invention, the ACT has the amino acid sequence shown in SEQ ID NO: 1.

In a still more particular embodiment, the ACT used in the process of the invention comprises the export signal for the specific machinery for secreting said toxin.

As has been previously explained, the inventors of the present invention have found that, when said ACT is bound to the outer membrane of a non-invasive bacterium, said non-invasive bacterium can be internalized and invade a eukaryotic cell.

Therefore, in another aspect, the present invention relates to a method, hereinafter method of the invention, for inducing the internalization of non-invasive bacteria comprising:
(a) incubating a non-invasive bacterium in the presence of an ACT, or of a functionally equivalent variant thereof, or transforming a non-invasive bacterium with a polynucleotide encoding an ACT, or a functionally variant thereof, and
(b) contacting the bacterium obtained in paragraph (a) with a eukaryotic cell culture.

In a particular embodiment of the method of the invention, the ACT has the amino acid sequence shown in SEQ ID NO: 1.

Additionally, in another particular embodiment, the bacterium of the method of the invention comprises
(i) a polynucleotide encoding a heterologous polypeptide of interest, or
(ii) a gene construct comprising a polynucleotide according to (i), or
(iii) a plasmid comprising a gene construct according to (ii) or
(iv) a heterologous polypeptide of interest.

The following example illustrates the invention and must not be considered as limiting the scope thereof.

### EXAMPLE 1

### Internalization of non-invasive bacteria in eukaryotic cells mediated by adenylate cyclase toxin (ACT)

### I. Materials and Methods

### 1.1 Obtaining and purification of ACT

ACT was produced in XL1-blue *E. coli* cells (Stratagene) transformed with plasmid pT7CACT1 (Martin et al., 2004. J. Bacteriol 186:3760-3765). The cell cultures (500 ml) in exponential phase were induced with 1 mM isopropyl-β-D-thio-galactoside (IPTG) for 3 hours. Then, the cells were sonicated and the inclusion bodies were extracted with 8 M urea, 50 mM Tris-HCl, pH 8 and 0.2 mM CaCl₂. The proteins were purified in successive ion exchange chromatographies in DEAE-Sepharose and Phenyl-Sepharose columns (Amersham Pharmacia Biotech) according to a known protocol (Sakamoto et al., 1992. JBC 267:13598-13602). In the final step, the proteins were flowed in 8 M urea, 50 mM Tris-HCl pH 8 and frozen at -20°C until their use.

### 1.2 Anchorage/adsorption of ACT to the bacterial membrane

The bacteria (*E. coli*) were grown in LB medium (Luria-Bertoni medium) for 12 hours. Then, they were centrifuged at 10,000 r.p.m. for 5 minutes and resuspended in buffer (20 mM Tris-HCl, 150 mM NaCl, 10 mM CaCl₂, pH 8) at an approximate concentration of 10⁸ bacteria/ml. 20 µg/ml of ACT were added to the bacterial suspension and the mixture was incubated at 37°C for 1 hour under constant stirring. In order to eliminate the ACT not bound to the bacteria, the suspension was centrifuged at 4,000 r.p.m. for 5 minutes and washed 3 times with the same buffer. To increase the efficiency of the binding of ACT to the bacterial surface, the process was repeated 5 times before the incubation with eukaryotic cells.

### 1.3 Quantification of cAMP

The intoxication activity of ACT was determined by means of quantifying the intracellular cAMP produced in Chinese hamster ovary (CHO) cells (LGC Prochem). After the incubation with ACT or with the bacteria coated with ACT, the CHO cells were homogenized in cold acidified ethanol and incubated for 5 minutes at ambient temperature. Then, the samples were centrifuged and the precipitates were washed with ethanol:water (2:1 v:v). The obtained supernatants were evaporated and the resulting pellets were resuspended in Tris-HCl/EDTA buffer to determine the cAMP by radioimmunoassay according to the manufacturer's instructions (Amersham Biosciences).

### 1.4 Assays of polymerization with actin-pyrene

The polymerization of G-actin [actin in globular form (G)] was determined by means of the fluorescence increase produced in the polymerization of the actin labeled with pyrene. The fluorescence was spectrofluorimetrically followed (365 nm excitation, 395 nm emission, 2.5 nm slits) in a FluoroMax-3 spectrofluorimeter (Horiba). G-actin labeled with pyrene and unlabeled G-actin were mixed in a 1:10 ratio in G-buffer (5 mM Tris-HCl pH 8.0, 0.1 mM ATP, 0.5 mM CaCl₂). The mixture was centrifuged at 150,000 g for 1 hour before its use. The polymerization of G-actin was started by adding 0.02 volumes of 50x polymerization start buffer (100 mM MgCl₂, 50 mM ATP, 2.5 M KCl) and the fluorescence in the samples was determined for 30 minutes at 37°C and under stirring.

### 1.5 Fluoresce microscopy

CHO cells were cultured until reaching confluence in Permanox Lab-Tek chambers in Dulbecco's modified essential medium (DMEM), supplemented with 10% (v/v) fetal bovine serum, L-glutamine and penicillin/streptomycin. ACT or the bacteria coated with ACT were added to the medium and co-incubated with CHO cells for 2 hours. Then, the cells were washed in phosphate buffered saline (PBS) pH 7.4, fixed in 3.7% formaldehyde and permeabilized in the presence of acetone for 3 minutes at -20°C.

To view the F-actin [actin in fibrous form (F)] and the DNA, the cells were stained with Alexa Fluor®488 phalloidin (Molecular Probes) and DAPI (Molecular Probes), respectively. Anti-ACT monoclonal primary (List Biological Laboratories, INC) and anti-mouse IgG secondary (Cell Signall Technology) antibodies labeled with Texas red® (Molecular Probes) were also used to detect the ACT bound to bacteria. The samples were viewed with a fluorescence microscope (Axioplan2, Zeiss) coupled to a digital camera (Axiocam NRc5, Zeiss). The images were processed with the Axovision 4 software.

### 1.6 Electron microscopy

For the preparation of the sections, the cells were grown until reaching confluence in 75 cm² flasks in DMEM medium, supplemented with 10% (v/v) fetal bovine serum, L-glutamine and penicillin/streptomycin. The bacteria incubated with ACT were added to the culture medium and co-incubated for 2 hours. Then, the cells were washed with phosphate buffered saline (PBS) pH 7.4, fixed in 2% glutaraldehyde and 0.1% tannic acid in 0.1 M sodium cacodylate. After the post-fixing in 1% OsO₄, the samples were dehydrated and embedded in Polarbed 814 epoxy resin (BioRad).

For the negative staining, the proteins were adsorbed in carbon coated copper grids for 1-2 minutes and washed in water before the negative staining (Harris, 1997, Royal Microscopical Society Microscopy Handbook No. 35. BIOS Scientific Publishers Ltd. Oxford, UK.) with 2% uranyl acetate pH 7.0.

The grids were examined in a Phillips CM100 transmission electron microscope at 80 kV.

### 1.7 Scanning electron microscope

The cells were grown until reaching sub-confluence in 13 mm round coverslips (Sarsted) in DMEM medium, supplemented with 10% (v/v) fetal bovine serum, L-glutamine and penicillin/streptomycin. The bacteria incubated with ACT were added to the culture medium and co-incubated for 2 hours. The cells were washed with PBS pH 7.4, fixed in 2% glutaraldehyde in 0.1 M sodium cacodylate, post-fixed in 1% OsO₄, successively dehydrated in ethanol and dried to the critical point. After the coating with gold, the samples were examined in a JSM-35C Jeol scanning microscope at 25 kV.

### 1.8 Dot blotting Assay

Different G-actin concentrations (0-50 µg) were carefully adsorbed on nitrocellulose membranes in successive applications of 2 µl. The membranes, once dry, were blocked for 3 hours with TBS supplemented with 5% BSA (bovine serum albumin). Then, they were incubated for 1 hour with different ACT concentrations (0-20 µg) in Tris-HCl buffer pH 8.0, 10 mM NaCl and 10 mM CaCl₂. The nitrocellulose membranes were washed three times with Tris-Buffered Saline Tween-20 (TBST), Tris-Buffered Saline (TBS) and 0.1% Tween and incubated with anti-ACT antibody [Commercial anti-RTX monoclonal antibody (Ref. 94D, BIOLOGICAL LABORATORIES INC)] for 1 hour at room temperature. After three new washings, the membranes incubated with secondary antibodies and the resulting bands were viewed by phosphorimaging. The experiments were repeated 3 times and the densitometric values are represented as the mean ± SD (standard deviation).

### 1.9 Bacterial invasion assays

In order to study the bacterial invasion the previously described gentamicin protection assay was used [Isberg, RR. and Falkow, SA. 1985. A single genetic locus encoded by *Yersinia pseudotuberculosis* permits invasion of cultured animal cells by *Escherichia coli* K-12. Nature. Vol.317(6034):262-264]. CHO cells seeded 36 hours before the assay in 96-well plates were left to grow until reaching confluence in DMEM medium with 10% fetal bovine serum. The cells were washed with antibiotic-free medium and incubated in medium without serum for 2 hours before the infection.

The CHO cells were infected with *E. coli* coated with ACT in a ratio of 100 bacteria per CHO cell. Then, the plates were incubated between 2 and 8 hours at 37°C, were washed and, subsequently, incubated for 1 hour in DMEM with gentamicin at a final concentration of 25 µg/ml to kill the extracellular bacteria. After 3 new washings with PBS, the cells were lysed in PBS with 0.1% triton X-100. The cell lysates were seeded in LB agar plates and the number of internalized bacteria was determined by counting colony forming units (cfu) after 24 hours of incubation at 37°C.

### II. Results

### 2.1 Effect of ACT on the intracellular formation of cAMP in CHO cells

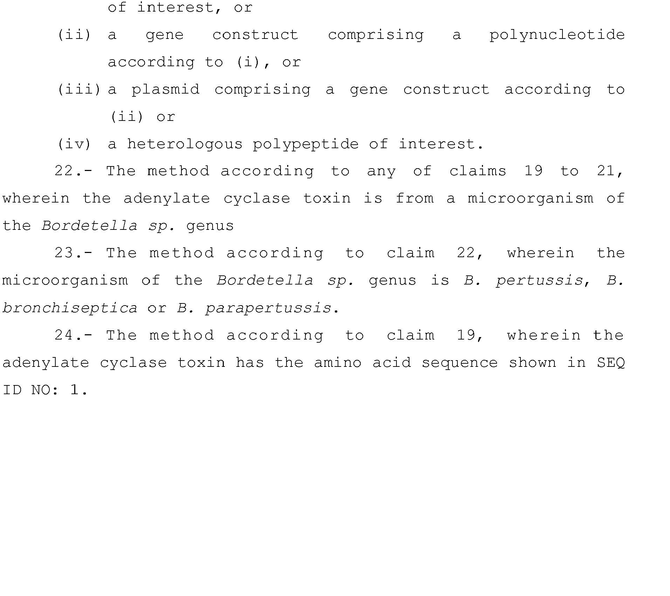

The capacity of ACT to intoxicate cells by means of increasing the intracellular cAMP levels was studied at high and low free ACT concentrations in the incubation medium. The addition of 20 µg/ml of ACT significantly increased the intracellular cAMP levels after 2 hours of incubation. These results coincide with previous studies in which it is demonstrated that ACT is capable of binding to and intoxicating different types of eukaryotic cells (Figure 1). Furthermore, the viability of said cells was affected and it was also observed that morphological changes appeared in the cell periphery such as the appearance of structures similar to pseudopodia. At low free ACT concentrations (2 µg/ml) neither the cell viability nor the intracellular cAMP levels were affected; however, morphological changes were also observed in the cells, although not as pronounced as and in the cells treated with high ACT concentrations. When CHO cells were co-incubated with the bacteria coated with ACT (20 µg/ml) results similar to those produced by the free ACT at low concentrations (2 µg/ml) were obtained (Figure 1). Likewise, the quantification of ACT bound to bacteria by means of Western-blot revealed that the amount of ACT bound to the outer membrane of the bacteria is 0.55 µg. Therefore, the structural modification of CHO cells is not caused by the alteration of the intracellular cAMP levels. These results suggest that the ACT could have a new activity responsible for the modifications observed in the morphology of CHO cells.

### 2.2 Effect of ACT on the cytoskeleton of CHO cells

In order to determine if the morphological modifications induced by ACT directly affected the cytoskeleton, actin fibers were viewed with Alexa Fluor®488 phalloidin once the treatments both with soluble ACT and with ACT bound to bacteria had ended. Unlike the control cells in which a very structured cytoskeleton was observed (Figure 2A), the treatment with free ACT at high concentrations resulted in a disorganization of the actin fibers. Furthermore, an accumulation of F-actin at the plasma membrane level was observed, as well as the formation of spicules or structures similar to pseudopodia (Figure 2B). The free ACT at low concentrations was also capable of inducing the formation of protrusions in the cell surface without drastically modifying the architecture of the cytoskeleton (Figure 2C). The formation of these structures similar to pseudopodia was also induced in CHO cells when co-incubated with the bacteria coated with ACT (Figure 2D). Similar effects on the cell cytoskeleton induced by pathogenic invasive bacteria such as *Listeria* or *Salmonella* have been previously described.

It has been previously demonstrated that *B. pertussis* is capable of invading epithelial cells and this effect has mainly been attributed to the filamentous hemagglutinin (FHA) associated with the bacterial membrane.

### 2.3 Effect of Cya associated with the E. coli membrane in cell invasion

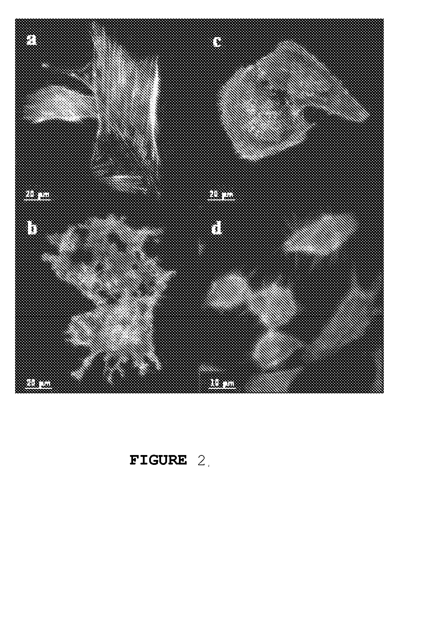

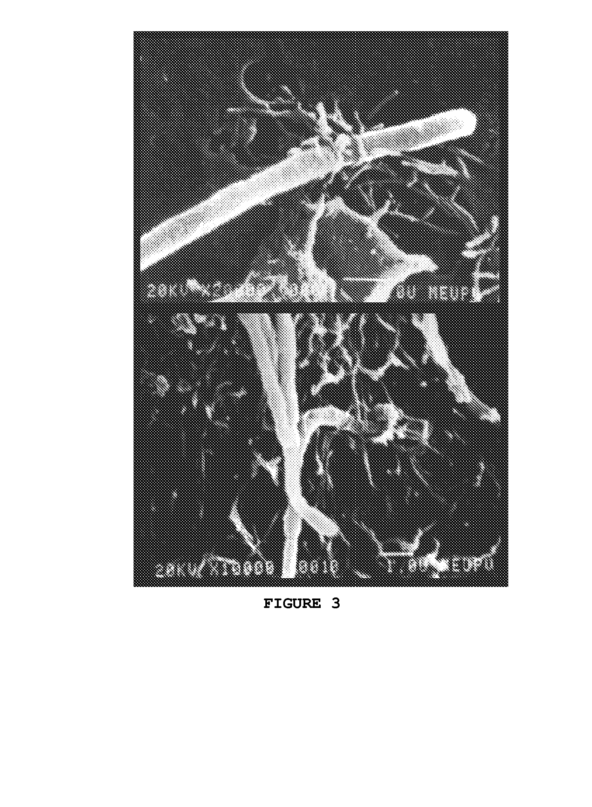

In order to prove if the ACT confers to the bacteria the capacity of invasion, CHO cells co-incubated with *E. coli* coated with ACT (20 µg/ml) were examined by electron microscopy. In the scanning electron microscope photos (Figure 3) it was observed how the CHO cells emitted prolongations similar to pseudopodia which strongly involved the bacteria coated with the ACT. In the transmission electron microscopy sections, different stages of the process which end with the internalization of the bacterium in the cell cytoplasm were observed. Thus, in a first moment, the contact of the bacterium with the cell was observed by means of a small prolongation which starts from the CHO cell (Figure 4A). In a more advanced step, a strong contact was established between the membranes of the bacteria and the membranes of the CHO cells (Figure 4B). Subsequently, the cell almost completely surrounded the bacterium similarly to phagocytosis (Figure 4C), and, finally, the bacteria inside the cell cytoplasm in structures similar to phagosomes could be observed (Figure 4D).

### 2.4 Interaction of ACT with G-actin

In order to prove if ACT has the capacity to bind to actin and confirm that the effects caused in the cytoskeleton are exerted by ACT, the capacity of ACT to bind to actin was studied by the dot blotting technique. In Figure 5 it can be verified that ACT was capable of interacting directly with G-actin previously immobilized in a nitrocellulose membrane. The negative results of binding to BSA confirm that the binding to actin is specific.

### 2.5 Polymerization of actin-G induced by ACT

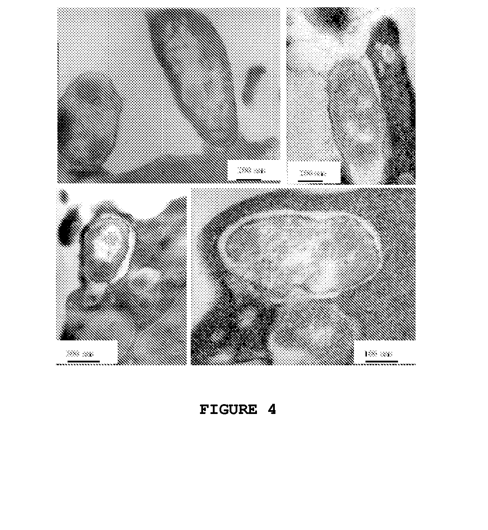

Below, the possibility that ACT could have a G-actin polymerizing effect was studied. To that end, control experiments of polymerization of G-actin labeled with pyrene were conducted. The addition of polymerization initiation buffer to the solution with G-actin caused a quick polymerization of the actin. This reaction was determined by the increase of the fluorescence emitted by pyrene when the actin polymerized. Then the effect of the polymerization of ACT was verified at different concentrations on G-actin. As can be seen in Figure 6, toxin causes a quick polymerizing effect which was furthermore dependent on the ACT concentration.

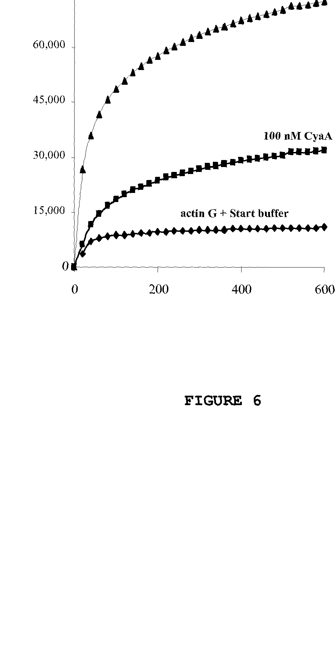

In order to prove that the effect exerted by the ACT was not due to an unspecific aggregation of the proteins, aliquots of the assays of polymerization were taken after 30 minutes and analyzed by negative staining in the electron microscope. The obtained results reveal the formation of long actin filaments in the preparations containing ACT (Figure 7).

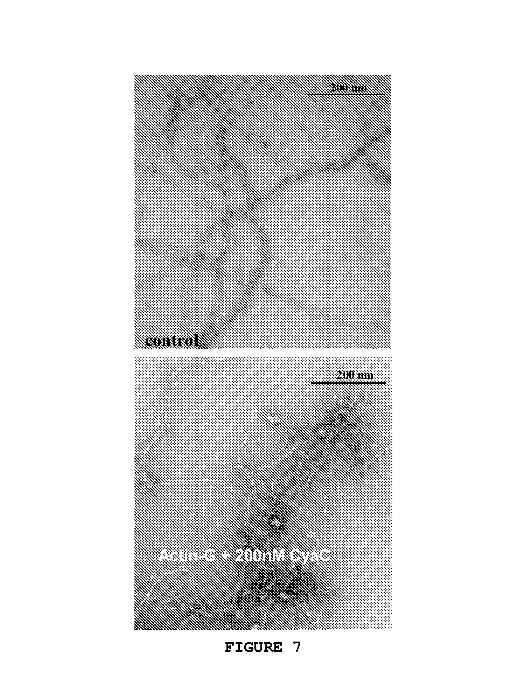

In order to prove if the toxin associated with the membrane of the bacteria preserved this actin polymerizing property, the bacteria coated with ACT were incubated with G-actin. After incubating for 2 hours, Alexa Fluor®488 phalloidin was added to fix F-actin and the sample was analyzed in the fluorescence microscope. In the control bacteria (*E. coli* without ACT) polymerization of actin was not found whereas in the assays performed with *E. coli* coated with ACT large halos of F-actin surrounding the bacteria were found (Figure 8). These experiments clearly show that ACT adhered to the bacterium preserves the G-actin polymerizing capacity observed in the in vitro polymerization experiments.

The results of this Example reveal that ACT induces a direct G-actin polymerization even when it remains associated with the membrane of the bacteria. This new function of the toxin would be responsible for the internalization of the bacteria coated with ACT in non-phagocytic cells.

### III. Conclusion

The results described in the present description show that purified ACT is capable of promoting the internalization of non-invasive bacteria in non-phagocytic mammal cells. This effect is mediated by a new function found by the inventors which causes the re-structuring of the cell cytoskeleton. This implies new expectations with possible applications in the techniques of gene vaccination and of presentation of heterologous antigens mediated by bacteria.

## Claims

1. Use of adenylate cyclase toxin, or a functionally equivalent variant thereof, as an inducing agent for the internalization of non-invasive bacteria in eukaryotic cells.

2. Use according to claim 1, wherein the adenylate cyclase toxin has the amino acid sequence shown in SEQ ID NO: 1.

3. Use according to claim 1 or 2, wherein the adenylate cyclase toxin is from a microorganism of the *Bordetella sp.* genus.

4. Use according to claim 3, wherein the microorganism of the *Bordetella sp.* genus is *B. pertussis, B. parapertussis* or *B. bronchiseptica.*

5. A non-invasive bacterium comprising an adenylate cyclase toxin or a functionally equivalent variant thereof bound to its membrane.

6. The bacterium according to claim 5, furthermore comprising
(i) a polynucleotide encoding a heterologous polypeptide of interest, or
(ii) a gene construct comprising a polynucleotide according to (i), or
(iii) a plasmid comprising a gene construct according to (ii) or
(iv) a heterologous polypeptide of interest.

7. The bacterium according to claim 5 or 6, wherein the adenylate cyclase toxin has the amino acid sequence shown in SEQ ID NO: 1.

8. The bacterium according to any of claims 5 to 7, wherein the adenylate cyclase toxin is from a microorganism of the *Bordetella sp.* genus.

9. The bacterium according to claim 8, wherein the microorganism of the *Bordetella sp.* genus is *B. pertussis, B. bronchiseptica* or *B. parapertussis.*

10. A pharmaceutical composition comprising a bacterium according to any of claims 5 to 9 and a pharmaceutically acceptable carrier.

11. A bacterium according to any of claims 5 to 9 for its use as a medicament.

12. Use of a bacterium according to any of claims 5 to 9 in the preparation of a vaccine.

13. Use according to claim 12 in the preparation of a vaccine for an immunization process by means of bactofection.

14. Use according to claim 12 in the preparation of a vaccine for an immunization process by means of an antigen-presenting bacterial system.

15. A process for obtaining a bacterium according to any of claims 5 to 9, comprising incubating a non-invasive bacterium with an adenylate cyclase toxin or a functionally equivalent variant thereof.

16. A process for obtaining a bacterium according to any of claims 5 to 9, comprising transforming a non-invasive bacterium with a polynucleotide encoding the adenylate cyclase toxin or a functionally equivalent variant thereof.

17. The process according to any of claims 15 and 16, wherein the adenylate cyclase toxin has the amino acid sequence shown in SEQ ID NO: 1.

18. The process according to any of claims 15 to 17, wherein the adenylate cyclase toxin comprises the export signal for the specific machinery for secreting said toxin.

19. A method for inducing the internalization of non-invasive bacteria in eukaryotic cells comprising:
(a) incubating the non-invasive bacterium in the presence of an adenylate cyclase toxin, or a functionally equivalent variant thereof, or transforming the non-invasive bacterium with a polynucleotide encoding an adenylate cyclase toxin, or a functionally equivalent variant thereof, and
(b) contacting the bacterium obtained in paragraph (a) with a eukaryotic cell culture.

20. The method according to claim 19, wherein the adenylate cyclase toxin has the amino acid sequence shown in SEQ ID NO: 1.

21. The method according to claim 19 or 20, wherein the bacterium comprises
(i) a polynucleotide encoding a heterologous polypeptide of interest, or
(ii) a gene construct comprising a polynucleotide according to (i), or
(iii) a plasmid comprising a gene construct according to (ii) or
(iv) a heterologous polypeptide of interest.

22. The method according to any of claims 19 to 21, wherein the adenylate cyclase toxin is from a microorganism of the *Bordetella sp.* genus

23. The method according to claim 22, wherein the microorganism of the *Bordetella sp.* genus is *B. pertussis, B. bronchiseptica* or *B. parapertussis.*

24. The method according to claim 19, wherein the adenylate cyclase toxin has the amino acid sequence shown in SEQ ID NO: 1.
